# FASCICULE DE BREVET EUROPEEN

(11) **EP 0 388 304 B1**
(45) Date de publication et mention de la délivrance du brevet: **28.09.1994**
(21) Numéro de dépôt: 90400697.0
(22) Date de dépôt: 15.03.1990
(51) Int. Cl.: C04B 41/00, C12P 3/00

(54) **Procédé de traitement biologique d'une surface artificielle**
Biologische Behandlung einer artifiziellen Oberfläche
Biological treatment of an artificial surface

(30) Priorité: 17.03.1989 FR 8903517
(43) Date de publication de la demande: 19.09.1990
(73) Titulaire: UNIVERSITE PIERRE ET MARIE CURIE PARIS VI, 75230 Paris Cedex 05 (FR)
(72) Inventeur: Adolphe, Jean Pierre, F-75017 Paris (FR); Loubiere, Jean-François, F-92300 Levallois-Perret (FR); Paradas, José, F-95310 Saint Ouen L'Aumone (FR); Soleilhavoup, François, F-93800 Epinay S/Seine (FR)
(74) Mandataire: Ahner, Francis

(56) Documents cités:
- EP-A- 0 127 721
- GB-A- 2 208 867
- US-A- 4 440 605
- US-A- 4 562 156
- CHEMICAL ABSTRACTS, vol. 106, no. 18, 4 mai 1987, page 274, abstract no. 142624n, Columbus, Ohio, US; V.V. BARANOV et al.: "Bacterial batch treatment in the manufacture of fine and structural ceramics",
- Orial et al. "La biominéralisation : un nouveau procédé de protection des pierres calcaires, application aux monuments histori ques" Rapport technique du Centre Expérimental de Recherches et d'Etudes du Batiment et des Travaux Publiques, Sept. 92, p. 26 dossier

## Description

La présente invention a pour objet un procédé de traitement microbiologique des surfaces artificielles notamment d'origine minérale, telles que les façades d'immeubles, les monuments ou des parois portant des signes rupestres, pour les rendre plus résistantes aux attaques du temps.

On sait que les matériaux de construction et notamment les matériaux de construction calcaires sont attaqués et dégradés par un grand nombre d'agents extérieurs, pluie, vent, microorganismes, poussières industrielles qui ne constituent qu'une sélection parmi les nombreux sévices que peuvent subir les constructions quelles qu'elles soient. Ces phénomènes de dégradation se sont accentués depuis un demi siècle en raison des pollutions urbaines et industrielles et notamment des phénomènes dits "de pluies acides".

Un des phénomènes à la base de ces attaques est lié à la pénétration de l'eau (eaux de pluie, rosée etc.) dans les matériaux de construction. D'une part cette imprégnation facilite l'érosion par des phénomènes liés aux variations de température, d'autre part, l'eau d'imprégnation sert de vecteur et de milieu d'action aux gaz acides dégagés par les activités humaines, en particulier l'anhydride sulfureux et l'anhydride nitrique. Les acides formés attaquent les matières acido-sensibles telles que les calcaires et les ciments.

A ces phénomènes physico-chimiques d'attaque acide et d'attaque mécanique (vent, gel), viennent s'ajouter les attaques d'origine biologique, notamment l'action des bactéries capables d'oxyder les sulfures et les sulfates sur les parois, telles que les Thiobacillus qui tendent à attaquer les matériaux contenant du calcium.

Les solutions proposées afin de protéger les matériaux de construction, en particulier les façades d'immeubles et les monuments, consistent la plupart du temps à appliquer sur la surface à protéger un revêtement de résine polymère imperméable, notamment des résines au silicone. Ce type de résine pénètre dans les pores de la roche et les bouche en rendant la surface imperméable.

Toutefois, ce procédé s'il donne de bons résultats à court terme, se révèle souvent catastrophique à la longue, lorsque de l'eau s'infiltre entre la partie rendue imperméable et la roche mère car les phénomènes physico-chimiques décrits précédemment vont se trouver amplifiés.

La présente invention se propose de fournir un procédé de traitement des surfaces des matériaux de construction qui permet d'en améliorer la résistance à l'érosion et à la pollution, tout en gardant une structure naturelle à la roche et/ou au matériau.

Un autre but de la présente invention est de fournir un procédé artificiel de formation de calcin maintenant une porosité des matériaux traités permettant des échanges gazeux entre le matériau et l'atmosphère ambiante.

Un autre but de la présente invention est de fournir un procédé du type précédent qui puisse être utilisé aussi bien sur les matériaux de construction neufs que sur les surfaces déjà altérées afin de les rendre à leur état initial.

Un autre but de la présente invention est de fournir un procédé de fabrication d'un calcin superficiel de couleur déterminée afin d'apparier la surface traitée à la couleur de la surface environnante.

La présente invention a également pour objet l'obtention d'une surface présentant l'aspect du calcin et qui peut être utilisée dans la décoration.

La présente invention concerne un procédé destiné à la protection et/ou au traitement d'une surface artificielle, de préférence d'origine minérale, grâce à un revêtement de surface, caractérisé en ce que ce revêtement de surface est réalisé in situ sur la surface artificielle en plaçant celle-ci au contact de microorganismes minéralisateurs dans un environnement autorisant la minéralisation.

Par surfaces artificielles, on entend désigner une surface qui a été travaillée, même de façon assez sommaire, ou qui porte des éléments que l'on souhaite conserver, ainsi la surface d'une pierre taillée sera considérée comme une surface artificielle, de même que les surfaces comportant des peintures pariétales.

La caractéristique de cette surface est que les microorganismes minéralisateurs peuvent s'y développer pour former une couche de calcin.

Le procédé selon l'invention préconise l'utilisation de microorganismes minéralisateurs, c'est-à-dire de microorganismes capables de produire des biominéralisations dans un environnement approprié.

Bien entendu, le procédé selon l'invention est plus particulièrement utilisable avec des microorganismes s'incrustant de carbonates de calcium, mais il est possible d'envisager d'autres types d'incrustation ou des co-incrustations. C'est pourquoi dans ce qui va suivre, on parlera plus particulièrement de calcin, à base de calcium, mais il est possible d'envisager d'autres revêtements tels que la barytine ou autres sels de baryum par exemple qui présentent une dureté plus élevée que les carbonates de calcium et qui résistent aux acides.

Les microorganismes minéralisateurs sont largement répandus, leur présence et leur relation avec les dépôts carbonatés tels que les tufs ou travertins ont déjà été décrits par Jean-Pierre ADOLPHE, notamment dans sa thèse intitulée "Observations et expérimentations géomicrobiologiques et physicochimiques des concrétionnements carbonatés continentaux actuelles et fossiles" (thèse de Doctorat d'Etat ès Sciences n° 81-34, Mémoires des Sciences de la Terre, Université Paris VI, 339 pages). Parmi ces microorganismes, il faut citer les algues, les champignons et surtout les bactéries.

Dans le procédé selon la présente invention, les microorganismes minéralisateurs seront plus particulièrement choisis parmi les familles bactériennes : Bacillaceae, de préférence dans le genre Bacillus, Pseudomonadaceae de préférence dans le genre Pseudomonas ou Enterobacteriaceae de préférence dans le genre Proteus.

Dans le procédé, il est possible d'utiliser des cultures pures d'une souche spécifique ou bien des mélanges.

A titre d'exemple spécifique de microorganismes utilisables dans le cadre de l'invention, il faut citer :
- Bacillus brevis,
- Bacillus licheniformis,
- Bacillus cereus,
- Bacillus megaterium
- Pseudomonas stutzerii
- Proteus mirabilis.

La liste des microorganismes n'est nullement limitative. D'autres microorganismes peuvent être sélectionnés de préférence à partir d'accumulations naturelles de bactéries calcifiées, des études ont en effet montré qu'il était possible de prélever ces microorganismes "fossilisés" ou incrustés par exemple, dans des dépôts calcaires, stalactites, concrétions des sols, mondmilch par exemple.

Les microorganismes selon l'invention doivent être mis en contact avec la surface à traiter dans des conditions autorisant la minéralisation, c'est-à-dire que ces microorganismes seront la plupart du temps sous forme de suspension dans un milieu liquide aqueux permettant la minéralisation.

Ce milieu liquide sera en général un milieu de culture adapté à la (ou aux) souche mise en oeuvre.

De préférence, on utilisera une (ou des) souche sauvage pour sa résistance et son pouvoir de compétition ; ces souches peuvent être soit trouvées dans des collections, soit prélevées en milieu naturel et isolées dans un laboratoire spécialisé. Dans ces conditions, le milieu utilisé sera la plupart du temps relativement simple, ces souches n'ayant pas en général de besoin spécifique, sauf éventuellement une source de carbone énergétique, en particulier dans le cas de souches prototrophes, un milieu synthétique pourra suffire. Néanmoins, il peut être utile de fournir à la souche des éléments plus assimilables ou plus énergétiques afin de favoriser son métabolisme, par exemple l'utilisation de bouillon de peptone, acides aminés, base purique et pyrimidinique et/ou vitamines.

Dans certains cas, il est possible d'envisager de modifier le milieu pour tenir compte d'une symbiose entre deux organismes.

Le pH du milieu devra être compatible avec la croissance du microorganisme et avec la nature du substrat.

Dans le procédé selon la présente invention, on utilisera de préférence une culture de microorganismes en phase exponentielle de croissance. Cette culture pourra donc être reconstituée avant l'utilisation afin d'être certains d'être en phase exponentielle. La durée de la pré-culture dépendra bien entendu de la souche mise en oeuvre et du milieu.

Dans le procédé selon la présente invention, il est préférable après la mise en présence des microorganismes et de la surface à traiter, de renouveller périodiquement le milieu de culture et éventuellement l'ensemencement pour être certains d'avoir un développement optimum du calcin.

Ainsi, on a mis en évidence que l'application périodique d'un milieu de culture, par exemple tous les jours après l'ensemencement, permettait d'observer la formation d'un calcin de structure très serrée et présentant une dureté supérieure à celle de la surface calcaire d'origine.

Dans le procédé selon la présente invention, la surface à traiter peut être immergée dans le milieu contenant les microorganismes ou bien il est possible de déposer sur la surface la suspension de microorganismes.

Le dépôt de la suspension peut être effectué par tout moyen approprié qui ne détruirait pas les microorganismes, application au pinceau ou au pulvérisateur par exemple.

La surface doit être traitée de préférence au refus mais également sans ruissellement.

De même, Il convient que le milieu nutritif soit réparti de manière homogène sur toute la paroi à traiter.

L'utilisation de ce procédé est particulièrement bien adapté au ravalement des façades avec l'avantage de reconstituer la pierre, ou le matériau de construction. En outre, ce procédé peut, si on le désire, colmater les joints et les petites fissures.

Le procédé par immersion dans un milieu de culture est applicable aux objets isolés ou pouvant l'être par démontage (statuaires, par exemple), de dimension modeste ou bien pour le prétraitement de matériau de construction, bloc de pierre par exemple.

Le procédé selon la présente invention peut être utilisé pour traiter des surfaces artificielles très diverses soit d'origine naturelle telle que pierres calcaires ou éruptives ou d'origine non naturelle telle que brique, tuile, béton, ciment, fibrociment, parpaing, bois et métaux.

Il est également possible de prévoir des surfaces artificielles telles que verre, caoutchouc et papier, ceci afin d'obtenir des surfaces destinées plus particulièrement à la décoration.

La mise en oeuvre du procédé selon la présente invention permet la formation rapide d'un calcin qui améliore significativement la résistance aux agressions atmosphériques et aux intempéries. Toutefois, comme cela a été indiqué précédemment, il est nécessaire que les conditions autorisent la minéralisation ; pour ce faire, il est nécessaire de prévoir l'apport d'ions calcium, notamment dans le milieu de culture. L'ion calcium peut au moins partiellement se trouver dans les produits de dégradation des pierres calcaires.

Ainsi, l'invention permet également de modifier l'aspect esthétique des surfaces traitées en utilisant des microorganismes minéralisant des ions présentant des formes colorées.

Enfin, on a trouvé de manière surprenante que la présence de ces organismes prévenaient le développement des bactéries et microorganismes responsables de la détérioration des matériaux de construction notamment Thiobacillus.

La présente invention est particulièrement bien adaptée aux matériaux de construction d'origine minérale, plus particulièrement aux matériaux de construction calcaires qu'ils soient naturels ou artificiels.

On peut citer notamment tous les matériaux de construction à base de carbonate de calcium.

On ne s'écartera pas de l'invention en substituant du baryum, partiellement ou en totalité, au calcium.

Les exemples non limitatifs suivants permettent à l'homme de metier de mieux comprendre l'intérêt de la présente invention.

### Exemple 1

Des échantillons d'un calcaire Jurassique bajocien à grains fins constitués de deux éprouvettes calcaires de 9X9X2 et deux éprouvettes calcaires de 10X10X4 ont été traités selon le procédé de la présente invention.

Le milieu nutritif était constitué de peptone 5g, glucose 10g, MgSO4 0,05 g, K2H PO4 0,5 g, NaCl 0,05 g, Ca(NO3)2 0,1g, K2 CO3 0,1g, MgCO3 0,1g, FeSO4 0,05g, ZnSO4 0,05g, NH4 NO3 0,5g (NH4)3 PO4 0,5, (NH4)2 SO4 0,5g, NaHCO3 0,05 g aux quantités suffisantes pour un litre. Le microorganisme utilisé était le Bacillus megaterium.

Une éprouvette de chaque dimension était complètement immergée cependant que l'autre éprouvette était partiellement immergée. La culture a été réalisée à 37°C en milieu aérobie. Les éprouvettes ont été soumises à un traitement dont la durée a varié de 15 à 20 jours. En fin d'expérience, il a eté constaté une grande homogénéité des effets de la minéralisation bactérienne. Les éprouvettes acquièrent dans leur ensemble des modifications d'aspect par rapport aux échantillons témoins non soumis au traitement. On constate notamment un changement de coloration et un colmatage superficiel de la roche. Il s'agit d'une véritable minéralisation carbonatée affectant la partie pelliculaire des échantillons. Cette biominéralisation soude les grains de la pierre lui conférant une meilleure cohésion et une plus grande dureté. Les éprouvettes traitées sont moins sensibles aux rayures à l'acier que les échantillons témoins. A la loupe binoculaire on constate sur des lames minces d'échantillons traités que cette minéralisation de surface produit un liseré de quelques dixièmes de millimètre. Ces observations sont confirmées au microscope photonique polarisant et microscope en fluorescence et au MEB. En outre, une patine a été obtenue lui donnant un aspect esthétique différent de celui du calcaire de départ.

### Exemple 2

Le procédé décrit à l'exemple 1 permet le traitement d'objets isolés. Le procédé décrit dans l'exemple en cause est utilisable sur des substrats en place.

Le milieu de culture utilisé est le suivant :

| | |
|---|---|
| peptone | 5 g |
| glucose | 10 g |
| MgSO4 | 0,05 g |
| K2 HPO4 | 0,5 g |
| NaCl | 0,05 g |
| Ca(NO3)2 | 0,1 g |
| K2 CO3 | 0,1 g |
| Mg CO3 | 0,1 g |
| Fe SO4 | 0,05 g |
| Zn SO4 | 0,05 g |
| NH4 NO3 | 0,5 g |
| (NH4)3 PO4 | 0,5 g |
| (NH4)2 SO4 | 0,5 g |
| Na2 CO3,H2O | 0,05 g |
| Eau q.s.p. | 1 l |

Ce milieu est stérilisé à l'autoclave pendant 20 minutes à 121°C. Après refroidissement il est ensemencé par les souches bactériennes selon l'invention.

Dans cet exemple, on ensemence directement avec un inoculum contenant Bacillus megaterium.

La surface à traiter du calcaire est nettoyée par tout moyen approprié, jeu de vapeur par exemple, dans certains cas des méthodes de nettoyage moins drastiques devront être utilisées.

Les souches sont mises en culture jusqu'à la phase exponentielle de croissance et la suspension est appliquée sur la surface à l'aide d'un pinceau.

On opère le revêtement de bas en haut en appliquant au refus mais en évitant le ruissellement. La paroi doit être imprégnée de façon homogène. Durant les deux semaines suivantes, on applique quotidiennement le milieu de culture seul. Au bout de deux semaines, on observe la formation d'une couche de calcin beaucoup moins poreux que la surface d'origine et beaucoup plus résistant à la rayure.

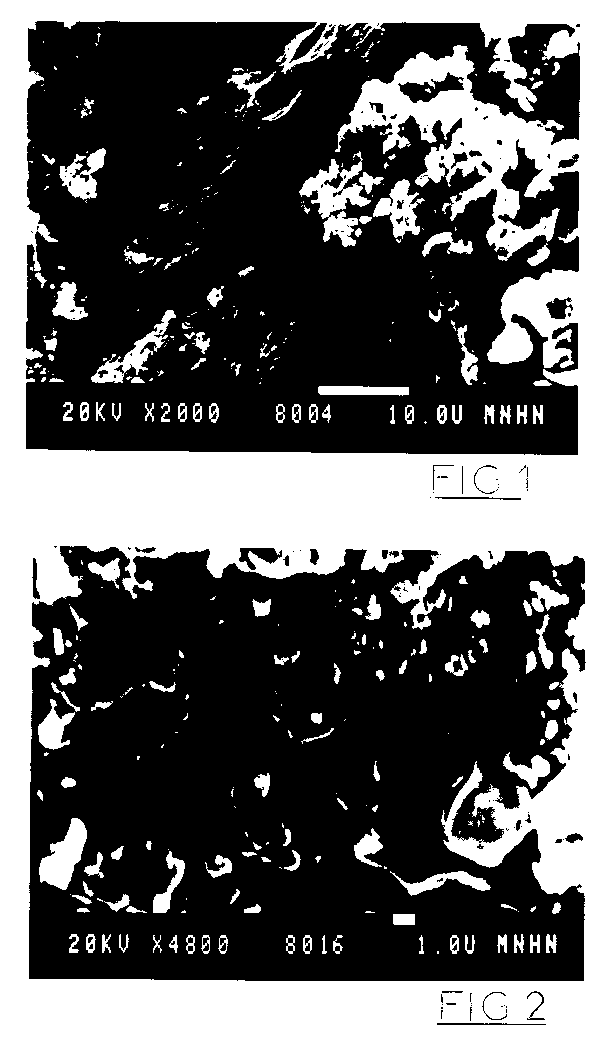

Les photographies ci-jointes, figures 1 et 2, permettent d'apprécier l'effet du procédé selon la présente invention :
- la figure 1 montre la surface de la pierre avant traitement : les éléments cristallins de la roche sont disjoints et il existe des microfissures, des micropores,
- la figure 2 montre la surface de la pierre après traitement : les éléments cristallins de la roche sont cimentés par les bactéries calcifiantes, les voiles bactériens minéralisés comblent les microfissures et les micropores.

Dans cet exemple, on a utilisé le microorganisme Bacillus megaterium, d'autres souches également disponibles dans des collections donnent de bons résultats, mais les prélèvements mixtes naturels peuvent être préférables.

## Revendications

1. Procédé destiné à la protection et/ou au traitement d'une surface artificielle de préférence d'origine minérale, grâce à un revêtement de surface, caractérisé par le fait que ce revêtement de surface est réalisé in situ sur la surface artificielle en plaçant celle-ci au contact de microorganismes minéralisateurs en phase exponentielle de croissance dans des conditions assurant la minéralisation.

2. Procédé selon la revendication 1, caractérisé par le fait que les microorganismes s'incrustent de carbonates de calcium.

3. Procédé selon les revendication 1 et 2 , caractérisé par le fait que le microorganisme minéralisateur est mis en contact avec la surface artificielle dans un milieu de culture.

4. Procédé selon l'une des revendications 1 à 3, caractérisé par le fait qu'après la mise en contact des microorganismes minéralisateurs avec la surface, on revêt cette surface régulièrement dans le temps avec un milieu de culture du microorganisme.

5. Procédé selon les revendications 1 à 4 , caractérisé par le fait que l'environnement autorise l'incrustation de carbonates de calcium.

6. Procédé selon la revendication 5 , caractérisé par le fait que l'environnement est constitué d'un milieu de culture du microorganisme contenant du calcium.

7. Procédé selon les revendications 1 à 6, caractérisé par le fait que les microorganismes sont choisis parmi les bactéries et les cyanobactéries.

8. Procédé selon la revendication 7, caractérisé par le fait que le microorganisme est une bactérie choisie dans les familles Bacillaceae, de préférence dans le genre Bacillus, Pseudomonadaceae de préférence dans le genre Pseudomonas ou Enterobacteriaceae de préférence dans le genre Proteus.

9. Procédé selon la revendication 8, caractérisé par le fait que les microorganismes sont choisis parmi les genres Bacillus et Pseudomonas.

10. Procédé selon l'une des revendications 1 à 9, caractérisé par le fait que les microorganismes sont sélectionnés à partir de prélevements effectués sur des accumulations de bactéries calcifiées.

11. Procédé selon l'une des revendications 1 à 10, caractérisé par le fait que la surface artificielle est constituée en tout ou partie d'une roche calcaire.

## Claims

1. A process intended for the protection and/or the treatment of an artificial surface, preferably of mineral origin, by virtue of a surface coating, in which this surface coating is produced in situ on the artificial surface by placing the latter in contact with mineralizing microorganisms in an exponential growth stage in conditions which ensure mineralization.

2. The process as claimed in claim 1, wherein the microorganisms are encrusted with calcium carbonates.

3. The process as claimed in claims 1 and 2, wherein the mineralizing microorganism is placed in contact with the artificial surface in a culture medium.

4. The process as claimed in one of claims 1 to 3, wherein, after the mineralizing microorganisms have been placed in contact with the surface, this surface is coated regularly in time with a culture medium for the microorganism.

5. The process as claimed in claims 1 to 4, wherein the environment permits the encrustation of calcium carbonates.

6. The process as claimed in claim 5, wherein the environment consists of a culture medium for the microorganism containing calcium.

7. The process as claimed in claims 1 to 6, wherein the microorganisms are chosen from bacteria and cyanobacteria.

8. The process as claimed in claim 7, wherein the microorganism is a bacterium chosen from the groups Bacillaceae, preferably from the genus Bacillus, Pseudomonadaceae, preferably from the genus Pseudomonas or Enterobacteriaceae, preferably from the genus Proteus.

9. The process as claimed in claim 8, wherein the microorganisms are chosen from the genera Bacillus and Pseudomonas.

10. The process as claimed in one of claims 1 to 9, wherein the microorganisms are chosen from samples taken from calcified accumulations of bacteria.

11. The process as claimed in one of claims 1 to 10, wherein the artificial surface consists wholly or partly of a calcareous rock.

## Patentansprüche

1. Verfahren zum Schützen und/oder Behandeln einer künstlichen Oberfläche vorzugsweise mineralischen Ursprungs durch Oberflächenbeschichtung, dadurch gekennzeichnet, daß diese Oberflächenbeschichtung in situ auf der künstlichen Oberfläche erfolgt, indem man sie mit mineralisierenden (mineralbildenden) Mikroorganismen in der exponentiellen Wachstumsphase unter Bedingungen, welche die Mineralisierung (Mineralbildung) gewährleisten, in Kontakt bringt.

2. Verfahren nach Anspruch 1, dadurch gekennzeichnet, daß die Mikroorganismen sich mit Calciumcarbonaten verkrusten (überziehen).

3. Verfahren nach den Ansprüchen 1 und 2, dadurch gekennzeichnet, daß der mineralbildende Mikroorganismus in einem Kulturmedium mit der künstlichen Oberfläche in Kontakt gebracht wird.

4. Verfahren nach einem der Ansprüche 1 bis 3, dadurch gekennzeichnet, daß man nach dem Kontaktieren der mineralbildenden Mikroorganismen mit der Oberfläche diese Oberfläche regelmäßig innerhalb der Zeit mit einem Kulturmedium des Mikroorganismus beschichtet.

5. Verfahren nach den Ansprüchen 1 bis 4, dadurch gekennzeichnet, daß die Umgebung die Inkrustation (Beschichtung) mit Calciumcarbonaten zuläßt.

6. Verfahren nach Anspruch 5, dadurch gekennzeichnet, daß die Umgebung besteht aus einem Medium zur Kultivierung des Mikroorganismus, das Calcium enthält.

7. Verfahren nach den Ansprüchen 1 bis 6, dadurch gekennzeichnet, daß die Mikroorganismen aus Bakterien und Cyanobakterien ausgewählt werden.

8. Verfahren nach Anspruch 7, dadurch gekennzeichnet, daß der Mikroorganismus ein Bakterium ist, das ausgewählt wird aus der Familie der Bacillaceae, vorzugsweise des Genus Bacillus, der Pseudomonadaceae, vorzugsweise des Genus Pseudomonas, oder der Enterobacteriaceae, vorzugsweise des Genus Proteus.

9. Verfahren nach Anspruch 8, dadurch gekennzeichnet, daß die Mikroorganismen unter den Genus Bacillus und Pseudomonas ausgewählt werden.

10. Verfahren nach einem der Ansprüche 1 bis 9, dadurch gekenzeichnet, daß die Mikroorganismen aus ab Anhäufungen von verkalt Bakterien abgezogenen Probestücken ausgewählt werden.

11. Verfahren nach einem der Ansprüche 1 bis 10, dadurch gekenzeichnet, daß die künstliche Oberfläche teilweise oder ganzlich aus einem Kalkstein konstitutiert ist.
